# EUROPEAN PATENT APPLICATION

(11) **EP 4 494 633 A1**
(43) Date of publication of application: **22.01.2025**
(21) Application number: 23386063.4
(22) Date of filing: 17.07.2023
(51) Int. Cl.: A61K 9/00, A61K 9/08, A61K 47/36, A61P 7/00

(54) **HYDROXYCARBAMIDE CONTAINING ORAL SOLUTION**

(71) Applicant: a Fine House S.A., 19441 Koropi (GR)
(72) Inventor: Liolios, Georgios, 15232 Chalandri Attica (GR)
(74) Representative: Roukounas, Dimitrios

(57) **Abstract**

An oral pharmaceutical solution comprising hydroxycarbamide as active ingredient and an aqueous carrier comprising a pharmaceutically acceptable buffer and 0.5 mg/ml to 2.4 mg/ml xanthan gum, wherein the pH of the solution is from 3.0 to 6.0.

## Description

### FIELD OF THE INVENTION

The present invention relates to an oral solution of a pharmaceutically acceptable salt of hydroxycarbamide.

### BACKGROUND OF THE INVENTION

Hydroxycarbamide, also known as hydroxyurea, is a compound used in the treatment of sickle-cell disease, essential thrombocythemia, chronic myelogenous leukemia, polycythemia vera, and cervical cancer. In sickle-cell disease it increases fetal hemoglobin and decreases the number of attacks.

Hydroxycarbamide has the following structure.

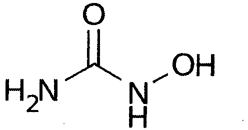

Patent US3119866 discloses compositions comprising hydroxycarbamide and a buffer as stabilizer. The compositions are preferably prepared by dissolving the buffer and hydroxycarbamide in water and evaporating off the water. The pH of the buffered solution is optimally about 5.5 to about 6.5. The solution is then frozen at once at about -30°C and lyophilized at 50-100 microns vacuum.

Patent application WO2019/220134 describes aqueous oral solutions comprising hydroxycarbamide and a pH adjuster being one of sodium hydroxide, potassium hydroxide, sodium bicarbonate, and sodium carbonate, or a mixture of one or more of these substances. According to WO2019/220134, the hydroxycarbamide solutions remain stable only when the solutions are initially controlled to have a pH of between 6.5 and 6.7 and the solutions are stored at temperature of 2°C to 8°C i.e. stored in a refrigerator.

In addition to the above, according to WO2019/220134 there is an unknown degradation impurity seen at RRT 0.44. The amount of the unknown impurity decreases with time when the pH of the solution is 6.2. Conversely, the amount of the unknown impurity increases significantly with time when the pH of the solutions is 6.6, especially when the solutions are stored at 25°C/60% RH.

The present invention overcomes the problems of the prior art and provides an oral pharmaceutical solution, comprising hydroxycarbamide, which exhibits excellent stability and extended lifetime.

### SUMMARY OF INVENTION

The present invention is directed to an oral pharmaceutical solution comprising hydroxycarbamide in association with a pharmaceutically acceptable aqueous carrier.

The oral pharmaceutical solution according to the invention comprises hydroxycarbamide as active ingredient and a pharmaceutically acceptable aqueous carrier comprising, a pharmaceutically acceptable buffer and 0.5 mg/ml to 2.4 mg/ml xanthan gum, wherein the pH of the solution is from 3.0 to 6.0.

With the present invention the stability of hydroxycarbamide in the oral solution is better than the stability of the oral solutions disclosed in the prior art. Thus, with the present invention the oral solution exhibits a longer shelf-life and can be stored at temperatures which are higher than those disclosed in the prior art.

### DETAILED DESCRIPTION OF THE INVENTION

The present invention provides a stable aqueous oral solution comprising hydroxycarbamide.

According to WO2019/220134 the main known degradation impurities of an aqueous oral solution comprising hydroxycarbamide are hydroxylamine (impurity A according to Ph. Eur. 11.0) and carbamoyloxyurea or (carbamoylamino) carbamate (CAS number: 4543-62-8, C₂H₅N₃O₃). In addition according to WO2019/220134 there is an unknown degradation impurity seen at Relative Retention Time (RRT) 0.44. The amount of the unknown impurity decreases with time when the pH of the solution is 6.2. Conversely, the amount of the unknown impurity increases significantly with time when the pH of the solutions is 6.6, especially when the solutions are stored at 25°C/60% RH.

The present inventors have surprisingly found that physicochemical stability of an oral solution comprising hydroxycarbamide is enhanced when the pH of the solution is from 3.0 to 6.0 and the concentration of xanthan gum in the solution is below 2.5 mg/ml.

More specifically it has been found that the unknown degradation impurity seen at RRT 0.44 in the oral solutions described in the prior art, is absent when the solution is stored at 25°C/60% RH for at least one month or at conditions of temperature 2°C - 8°C for at least 9 months, when the pH of the solution is from 3.0 to 6.0 and the concentration of xanthan gum in the solution is below 2.5 mg/ml.

The oral pharmaceutical solution according to the invention comprises hydroxycarbamide as active ingredient and a pharmaceutically acceptable aqueous carrier comprising a pharmaceutically acceptable buffer and 0.5 mg/ml to 2.4 mg/ml xanthan gum, wherein the pH of the solution is from 3.0 to 6.0.

Preferably, the concentration of xanthan gum is from 1.0 mg/ml to 2.2 mg/ml.

Preferably, the oral solution comprises from 80 mg/ml to 120 mg/ml hydroxycarbamide. More preferably, the oral solution comprises from 90 mg/ml to 110 mg/ml hydroxycarbamide.

The term "mg/ml" as used throughout the present application means mg of the active ingredient or of an excipient per 1 ml of the oral solution.

Preferably the pH of the solution is from 3.5 to 5.5. More preferably, the pH of the solution is from 4.0 to 5.0.

Any pharmaceutically acceptable system which acts as a buffer in the pH region of the invention can be used in the oral pharmaceutical solution according to the invention. Examples of a buffering agent include but are not limited to ascorbic acid, acetic acid, tartaric acid, citric acid monohydrate, sodium citrate, potassium citrate, acetic acid, sodium acetate, sodium hydrogen phosphate, sodium dihydrogen phosphate, calcium hydrogen phosphate, calcium dihydrogen phosphate, or a mixture thereof.

The hydroxycarbamide-containing oral solution according to the invention exhibits excellent physicochemical stability at room temperature (25°C) and 60% relative humidity (RH) for at least 1 month.

The hydroxycarbamide-containing oral solution according to the invention exhibits excellent physicochemical stability at a temperature of 2°C - 8°C for at least 9 months.

The oral solution of hydroxycarbamide, according to the invention may be supplied as monodose or multidose preparation. Preferably, the oral solution according to the invention is supplied as a multidose preparation.

Each dose from a multidose sealed vial, can be administered by means of a device suitable for measuring the prescribed volume. The device is typically a spoon or a cup for volumes of 5 ml or multiples thereof, or an oral syringe for other volumes.

The oral solutions according to the invention may also optionally contain additional excipients commonly used in preparing oral liquid compositions, such as antimicrobial preservatives, sweeteners and flavouring agents.

Antimicrobial preservatives include but are not limited to benzoic acid, boric acid, sorbic acid and salts thereof, sodium benzoate, benzyl alcohol, parahydroxy benzoic acid, methyl, ethyl or propyl parahydroxy benzoate and their salts, or mixtures thereof.

Sweeteners include but are not limited to sucralose, aspartame, acesulfame-K, thaumatin, mogroside, saccharin and salts thereof, sodium cyclamate, glucose, sucrose, lactose, fructose, erythritol, glycyrrhizin, monosodium glycyrrhizinate, monoamonium glycyrrhizinate, dextrose or mixtures thereof.

Flavouring agents include but are not limited to fruit flavours such as orange, banana, strawberry, cherry, wild cherry, lemon and the like and other flavourings, such as cardamom, anise, mint, menthol, vanillin, bubble gum or mixtures thereof.

The oral pharmaceutical solution of the present invention may be prepared using methods well known in the art and using regular manufacturing equipment.

For example, it may be prepared using the following process:
The active substance and the excipients are weighed. Purified water is added into a vessel. Hydroxycarbamide and the xanthan gum, are successively dissolved in purified water under stirring. A buffer solution, prepared in a different vessel, is added under continuous stirring until hydroxycarbamide is completely dissolved. Preservative, if present, is also added under continuous stirring until complete dissolution. Flavour and the remaining excipients, if present, are successively added under continuous stirring, until complete dissolution. The pH of the solution is adjusted with a quantity of the buffer solution to the desired value. Finally, the volume is adjusted with purified water. The final solution is filled in the container (e.g. glass bottles).

### EXAMPLES

### EXAMPLE 1

The present example shows the stability of a composition according to a present invention and the improvement over the composition of Table 6 of WO2019/220134.

The hydroxycarbamide solutions were prepared in the following manner:
Purified water was added into a vessel. Hydroxycarbamide and xanthan gum were successively dissolved in purified water under stirring. A buffer solution (or a NaOH solution), prepared in a different vessel, was added under continuous stirring until hydroxycarbamide was completely dissolved. Methyl paraben was also added under continuous stirring until complete dissolution. Flavour and sucralose were successively added under continuous stirring, until complete dissolution. The pH of the solution was adjusted with a quantity of the buffer solution. Finally, the volume was adjusted with purified water. The final solution was filled in 50 ml amber type class III glass bottles.

The compositions were stored at 25°C temperature and 60% relative humidity for a total period of two months and at a temperature of 2 °C to 8°C (refrigeration) for a total period of two months. Quantification of hydroxycarbamide and its impurities in the compositions was performed by HPLC.

**Table 1 Composition of Hydroxycarbamide oral solution 100 mg/ml (Trial 1)**

| **Component** | **Function** | **Trial 1 (pH 4.5)** |
|---|---|---|
| Hydroxycarbamide | API | 100 mg/ml |
| Citric acid 0.1M | Buffering agent | 1.0 mg/ml |
| Sodium citrate 0.1M | Buffering agent | 1.2 mg/ml |
| Xanthan gum | Thickening agent | 2.1 mg/ml |
| Methyl paraben | Preservative | 0.5 mg/ml |
| Sucralose | Sweetener | 2.0 mg/ml |
| Strawberry flavour | Flavouring agent | 1.0 mg/ml |
| Sodium citrate/ Citric acid 10% | pH adjusting agent | Qs to pH 4.5 |
| Purified water | Diluent | Qs to 1 ml |

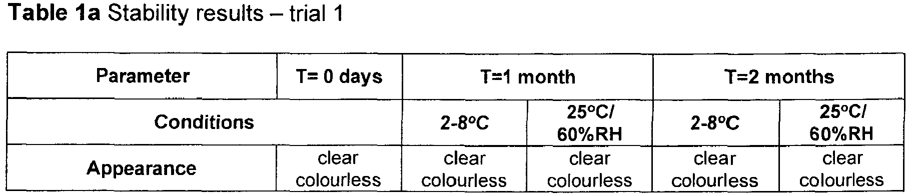

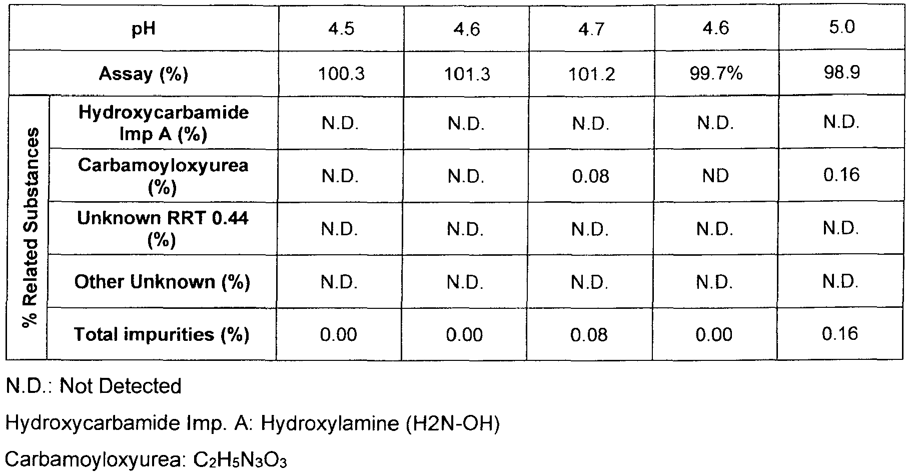

Table 1a shows that the unknown degradation impurity seen at RRT 0.44 in the oral solutions described in Table 6 of WO2019/220134, is absent when a solution having a pH equal to 4.5 and comprising 2.1 mg/ml xanthan gum is stored at 25°C/60% RH for one month, or at 2°C - 8°C for two months. On the other hand, Table 2a shows that in the composition of Table 2 (which is the composition of Table 6 of WO2019/220134), the unknown impurity at RRT 0.44 appears already after storage of 1 month at 25°C/60%RH. Furthermore, Tables 1a and 2a show that the amount of carbamoyloxyurea in the composition of the present invention is significantly lower than in the composition of WO2019/220134, after storage at 2°C - 8°C and after storage at 25°C/60%RH.

**Table 2 Composition of Table 6 of WO2019/220134**

| **Component** | **Function** | **Table 6 of** WO 20191220134 |
|---|---|---|
| Hydroxycarbamide | API | 100 mg/ml (10% w/v) |
| Xanthan gum | Thickening agent | 4.0 mg/ml (0.4% w/v) |
| Methyl paraben | Preservative | 0.5 mg/ml (0.05% w/v) |
| Sucralose | Sweetener | 2.0 mg/ml (0.2% w/v) |
| Strawberry flavour | Flavouring agent | 1.0 mg/ml (0.1 % w/v) |
| 1 M NaOH solution | pH adjusting agent | Qs to pH 6.6 (∼0.7% v/v) |
| Purified water | Diluent | Qs to 1 ml |

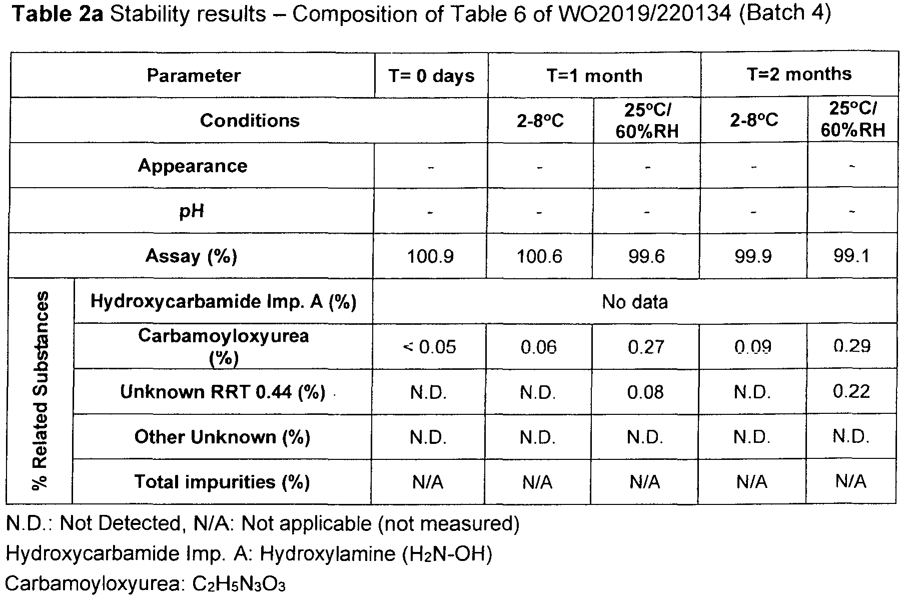

### EXAMPLE 2

The present example shows the stability of a composition according to the present invention comprising 1.1 mg/ml xanthan gum.

The hydroxycarbamide solution shown in Table 3 was prepared in the following manner:
Purified water was added into a vessel. Hydroxycarbamide and xanthan gum were successively dissolved in purified water under stirring. A buffer solution (sodium citrate/ citric acid), prepared in a different vessel, was added under continuous stirring until hydroxycarbamide was completely dissolved. Methyl paraben was also added under continuous stirring until complete dissolution. Flavour and sucralose were successively added under continuous stirring, until complete dissolution. The pH of the solution was adjusted with a quantity of the buffer solution. Finally, the volume was adjusted with purified water. The final solution was filled in 50 ml amber type class III glass bottles.

The composition was stored at 25°C temperature and 60% relative humidity for a total period of one month and at a temperature of 2°C to 8°C (refrigeration) for a total period of nine months. Quantification of hydroxycarbamide and its impurities in the composition was performed by HPLC.

**Table 3 Composition of Hydroxycarbamide oral solution 100 mg/ml**

| **Component** | **Function** | **Trial 2 (pH 4.5)** |
|---|---|---|
| Hydroxycarbamide | API | 100 mg/ml |
| Citric acid 0.1M | Buffering agent | 1.0 mg/ml |
| Sodium citrate 0.1M | Buffering agent | 1.2 mg/ml |
| Xanthan gum | Thickening agent | 1.1 mg/ml |
| Methyl paraben | Preservative | 0.5 mg/ml |
| Sucralose | Sweetener | 2.0 mg/ml |
| Strawberry flavour | Flavouring agent | 1.0 mg/ml |
| Sodium citrate/ Citric acid 10% | pH adjusting agent | Qs to pH 4.5 |
| Purified water | Diluent | Qs to 1 ml |

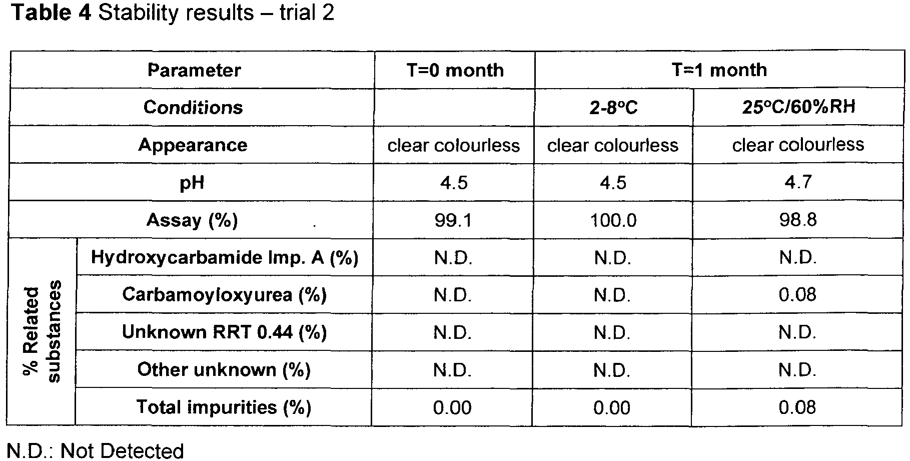

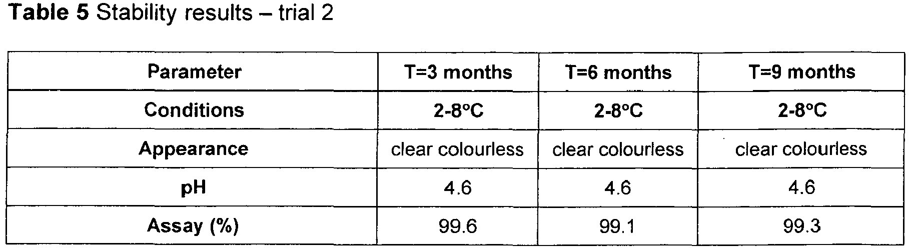

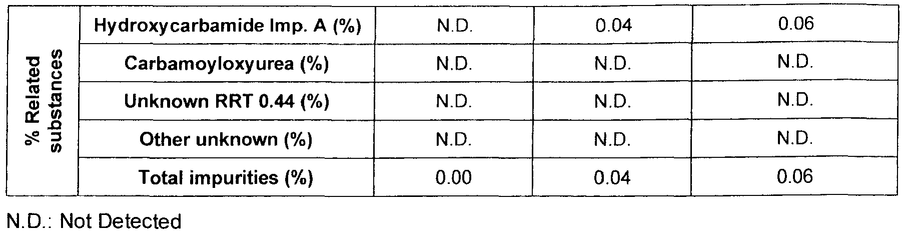

The results of Table 4 and Table 5 show that the unknown degradation impurity seen at RRT 0.44 in the oral solutions described in Table 6 of WO 2019/220134 does not appear in a composition according to the present invention. Furthermore, the results show that the amount of carbamoyloxyurea in the composition of the present invention is significantly lower than in the composition of WO2019/220134, after storage at 2°C - 8°C and after storage at 25°C/60%RH.

## Claims

1. An oral pharmaceutical solution comprising hydroxycarbamide as active ingredient and a pharmaceutically acceptable aqueous carrier comprising a pharmaceutically acceptable buffer and 0.5 mg/ml to 2.4 mg/ml xanthan gum, wherein the pH of the solution is from 3.0 to 6.0.

2. The oral pharmaceutical solution according to claim 1, wherein the concentration of xanthan gum in the solution is from 1.0 mg/ml to 2.2 mg/ml.

3. The oral pharmaceutical solution according to any one of the preceding claims, wherein the concentration of hydroxycarbamide in solution is from 80 mg/ml to 120 mg/ml.

4. The oral pharmaceutical solution according to any one of the preceding claims, wherein the concentration of hydroxycarbamide in the solution is from 90 mg/ml to 110 mg/ml.

5. The oral pharmaceutical solution according to any one of the preceding claims, wherein the pH of the solution is from 3.5 to 5.5.

6. The oral pharmaceutical solution according to any one of the preceding claims, wherein the pH of the solution is from 4.0 to 5.0.

7. The oral pharmaceutical solution according to any one of the preceding claims, further comprising an excipient selected from the group consisting of an antimicrobial preservative, a flavouring agent, a sweetener, and a mixture thereof.

8. The oral pharmaceutical solution according to claim 7, wherein the antimicrobial preservative is selected from the group consisting of benzoic acid, boric acid, sorbic acid and a salt thereof, sodium benzoate, benzyl alcohol, parahydroxy benzoic acid, methyl, ethyl or propyl parahydroxy benzoate and a salt thereof, and mixtures thereof.

9. The oral pharmaceutical solution according to claim 7 or 8, wherein the antimicrobial preservative is methyl, ethyl, or propyl parahydroxy benzoate, or a salt thereof.

10. The oral pharmaceutical solution according to any one of claims 7 to 9, wherein the flavouring agent is selected from the group consisting of orange, banana, strawberry, cherry, wild cherry, lemon cardamom, anise, mint, menthol, vanillin, bubble gum flavour, and mixtures thereof.

11. The oral pharmaceutical solution according to any one of claims 7 to 10, wherein the flavouring agent is strawberry flavour.

12. The oral pharmaceutical solution according to any one of claims 7 to 11, wherein the sweetener is selected from the group consisting of sucralose, aspartame, acesulfame-K, thaumatin, mogroside, saccharin and salts thereof, sodium cyclamate, glucose, sucrose, lactose, fructose, erythritol, glycyrrhizin, monosodium glycyrrhizinate, monoamonium glycyrrhizinate, dextrose, and mixtures thereof.

13. The oral pharmaceutical solution according to any one of claims 7 to 12, wherein the sweetener is sucralose.
